# EUROPÄISCHE PATENTSCHRIFT

(11) **EP 2 609 410 B1**
(45) Veröffentlichungstag und Bekanntmachung des Hinweises auf die Patenterteilung: **18.04.2018**
(21) Anmeldenummer: 11749737.0
(22) Anmeldetag: 24.08.2011
(51) Int. Cl.: G01M 3/28, G01M 3/38, G01N 9/24

(54) **VERFAHREN ZUM DETEKTIEREN VON LECKAGEN, SYSTEM UND MEDIZINTECHNISCHE BEHANDLUNGSVORRICHTUNG**
METHOD FOR DETECTING LEAKS, SYSTEM, AND MEDICAL TREATING DEVICE
PROCÉDÉ DE DÉTECTION DE FUITES, SYSTÈME ET DISPOSITIF DE TRAITEMENT UTILISÉ EN TECHNIQUE MÉDICALE

(30) Priorität: 25.08.2010 DE 102010035498
(43) Veröffentlichungstag der Anmeldung: 03.07.2013
(73) Patentinhaber: Fresenius Medical Care Deutschland GmbH, 61352 Bad Homburg (DE)
(72) Erfinder: WIKTOR, Christoph, 63571 Gelnhausen (DE)
(74) Vertreter: Bobbert & Partner Patentanwälte PartmbB
(86) Internationale Anmeldenummer: PCT/EP2011/004241
(87) Internationale Veröffentlichungsnummer: WO 2012/025225

(56) Entgegenhaltungen:
- EP-A1- 1 666 864
- US-B1- 6 740 036

## Beschreibung

Die vorliegende Erfindung betrifft ein Verfahren zum Detektieren von Leckagen gemäß Anspruch 1. Sie betrifft ferner ein System gemäß Anspruch 10 sowie eine medizintechnische Behandlungsvorrichtung gemäß Anspruch 15.

Das Auftreten von Leckagen in Systemen, welche medizinische Fluide führen, kann regelmäßig problematisch bis gefährlich sein. Eine frühzeitige Detektion derartiger Leckagen ist daher von großer Bedeutung.

EP 1 666 864 A1 offenbart einen Leckdetektor mit einem Drucksensor. US 6 740 036 B1 offenbart eine Vorrichtung mit einem Sensor zur Bestimmung von Blutparameter eines Patienten.

Eine Aufgabe der vorliegenden Erfindung ist, ein zur Detektion von Leckagen geeignetes Verfahren vorzuschlagen. Ferner sollen ein geeignetes System sowie eine medizintechnische Behandlungsvorrichtung angegeben werden.

Die erfindungsgemäße Aufgabe wird durch ein Verfahren mit den Merkmalen des Anspruchs 1 gelöst. Sie wird ferner durch ein System mit den Merkmalen des Anspruchs 10 sowie eine medizintechnische oder medizinische Behandlungsvorrichtung mit den Merkmalen des Anspruchs 15 gelöst.

Alle mit dem erfindungsgemäßen Verfahren erzielbaren Vorteile lassen sich ungeschmälert auch mit dem System und/oder der medizintechnischen Behandlungsvorrichtung erzielen.

Das erfindungsgemäße Verfahren ist geeignet und vorgesehen zur Detektion von Leckagen eines ein medizinisches Fluid führenden Systems, insbesondere stromaufwärts einer Absperreinrichtung des Systems, welches wenigstens die folgenden Komponenten aufweist: wenigstens eine Fördereinrichtung, eine Absperreinrichtung und einen das medizinische Fluid führenden Abschnitt stromaufwärts der Absperreinrichtung. Die Fördereinrichtung dient dazu, das Fluid durch den Abschnitt des Systems hindurch zu fördern. Dies ist wenigstens in Richtung zur Absperreinrichtung möglich. Darüber hinaus kann die Fördereinrichtung optional ergänzend zum Fördern auch in entgegengesetzter Richtung ausgestaltet oder angeordnet oder konfiguriert sein.

Das erfindungsgemäße Verfahren umfasst das Unterbrechen oder Verringern eines Fluidstroms des medizinischen Fluids durch den Abschnitt hindurch oder aus diesem heraus während einer ersten Förderanstrengung der Fördereinrichtung. Zum Unterbrechen oder Verringern wird die Absperreinrichtung entsprechend eingestellt, z. B. geschlossen. In diesem Einstellungszustand wird im Abschnitt ein erster Förderzustand erzielt.

Ferner umfasst das erfindungsgemäße Verfahren das Erzielen eines zweiten Förderzustands im Abschnitt durch Verändern der Förderanstrengung der Fördereinrichtung; aus der ersten Förderanstrengung wird eine zweite Förderanstrengung.

In einem weiteren Schritt umfasst das erfindungsgemäße Verfahren das Emittieren wenigstens eines Signals mittels einer Signalsendeeinrichtung - sowohl im ersten als auch im zweiten Förderzustand - in den Abschnitt des Systems hinein (messbar z. B. innerhalb des Abschnitts mittels eines darin vorliegenden Sensors) und ggf. durch diesen hindurch (messbar z. B. als Transmission auf der Seite des Abschnitts, welcher der Signaleinfallseite gegenüberliegt, oder als Reflexion auf der Seite des Abschnitts, von welcher aus das Signal emittiert wurde).

Derjenige Anteil des emittierten Signals, welcher im ersten Förderzustand den Abschnitt wieder verlässt (oder in diesen eindringt und innerhalb des Abschnitts von der Signalempfangseinrichtung empfangen wird), wird dabei mittels einer Signalempfangseinrichtung empfangen und im Folgenden als ein erster Anteil bezeichnet. Analog wird der Anteil des emittierten Signals, welches im zweiten Förderzustand den Abschnitt wieder verlässt (oder in diesen eindringt und innerhalb des Abschnitts von der Signalempfangseinrichtung empfangen wird) mittels der Signalempfangseinrichtung empfangen und im Folgenden als ein zweiter Anteil bezeichnet.

Anschließend kann in einem weiteren Schritt des erfindungsgemäßen Verfahrens anhand einer Auswertung des ersten Anteils in Bezug auf den zweiten Anteil eine Aussage darüber getroffen werden, ob eine Leckage vorliegt oder nicht.

Das erfindungsgemäße System weist wenigstens eine Steuereinrichtung auf, welche geeignet und/oder konfiguriert und/oder vorgesehen ist zum Ausführen des erfindungsgemäßen Verfahrens.

Die erfindungsgemäße medizintechnische oder medizinische Behandlungsvorrichtung ist vorgesehen zu ihrer Verbindung, oder ist verbunden, mit wenigstens einem erfindungsgemäßen System. Daneben oder stattdessen ist die erfindungsgemäße Behandlungsvorrichtung in bestimmten Ausführungsformen derselben vorgesehen zum Durchführen wenigstens eines erfindungsgemäßen Verfahrens.

Vorteilhafte Weiterbildungen der vorliegenden Erfindung sind jeweils Gegenstand der Unteransprüche.

Erfindungsgemäße Ausführungsformen können einige oder alle der folgenden Merkmale in beliebiger Kombination aufweisen.

In manchen erfindungsgemäßen Ausführungsformen umfasst das emittierte Signal Ultraschallwellen oder besteht hieraus. Dabei emittiert die Signalsendeeinrichtung Ultraschallwellen, und die Signalempfangseinrichtung detektiert Ultraschallwellen. Das erfindungsgemäße System kann entsprechend ausgestaltet sein.

In bestimmten erfindungsgemäßen Ausführungsformen sind die Signalsendeeinrichtung und/oder die Signalempfangseinrichtung Piezokristalle oder weisen solche auf.

In manchen erfindungsgemäßen Ausführungsformen umfasst das emittierte Signal Strahlung oder besteht hieraus. Dabei ist die Signalsendeeinrichtung eine Strahlungsquelle zum Emittieren von Strahlung, und die Signalempfangseinrichtung ist ein Strahlungsempfänger zum Empfangen oder Detektieren von Strahlung. Das erfindungsgemäße System kann entsprechend ausgestaltet sein.

In bestimmten erfindungsgemäßen Ausführungsformen sind die Signalsendeeinrichtung und die Signalempfangseinrichtung ein und dieselbe Einrichtung. Diese Einrichtung dient somit sowohl zum Senden als auch zum Empfangen des betreffenden Signals. Diese erfindungsgemäße Ausgestaltung kann ausgeführt werden ungeachtet der Art des zu übertragenden und zu empfangenden Signals. Eine gemeinsame Verkörperung von Signalsendeeinrichtung und Signalempfangseinrichtung als eine einzige Einrichtung, welche nach Umschalten oder Umkehr des Wirkprinzips zwischen Senden und Empfangen variieren kann, ist - ebenso wie eine Verkörperung von Signalsendeeinrichtung und Signalempfangseinrichtung als getrennte Einrichtungen, welche aber in einem gemeinsamen Gehäuse vorliegen - sowohl bei der Verwendung von Ultraschall als auch bei der Verwendung von anderen Signalarten Gegenstand von Ausführungsformen der vorliegenden Erfindung.

Die vorliegende Erfindung kann, ohne hierauf jedoch beschränkt zu sein, vorteilhaft dazu eingesetzt werden, Leckagen in einem extrakorporalen Blutkreislauf und/oder undichte Konnektoren an einer Blutbehandlungseinrichtung wie z. B. einem Dialysator aufzudecken.

Der Begriff "System", wie er hierin verwendet wird, bezeichnet in bestimmten Ausführungsformen der vorliegenden Erfindung ein (medizin-)technisches System - im Gegensatz zu einem Gefäßsystem eines Patienten.

In bestimmten Ausführungsformen der vorliegenden Erfindung weist das System eine zur Führung von Fluiden geeignete und/oder vorgesehene Anordnung verschiedener Bauelemente, wie beispielsweise Leitungen, Schläuche, Kanäle, strömungsfördernden und/oder strömungsverringernden Elementen, Zuführungs- und/oder Ableitungseinrichtungen und dergleichen, auf. In bestimmten Ausführungsformen ist das System beispielsweise, ohne jedoch hierauf beschränkt zu sein, als Schlauchsystem, wie ein extrakorporaler Blutkreislauf ausgestaltet.

Das System ist ausgestaltet und/oder vorgesehen, wenigstens ein medizinisches Fluid mittels eines im System enthaltenen Abschnitts, wie einem Inneren, z. B. einem Leitungsinneren, desselben aufzunehmen und/oder zu leiten.

Der Begriff "medizinisches Fluid", wie er hierin verwendet wird, bezeichnet in bestimmten erfindungsgemäßen Ausführungsformen ein Fluid, welches extrakorporal vorliegt oder strömt und vorzugsweise - z. B. im Rahmen einer extrakorporalen Blutbehandlung - zu behandeln ist.

Das medizinische Fluid ist in manchen erfindungsgemäßen Ausführungsformen eine Flüssigkeit wie Blut, Dialysat, Substituat, Medikamentenlösung(en), ein Gas oder eine Kombinationen oder Mischung dergleichen.

Der Begriff "Abschnitt", wie er hierin verwendet wird, bezeichnet in bestimmten Ausführungsformen der vorliegenden Erfindung einen Teil des Systems, in welchem die zu detektierende Leckage auftritt oder auszuschließen ist.

Der Begriff "Leckage", wie er hierin verwendet wird, bezeichnet ein Leck, eine - unerwünschte - Öffnung, eine Undichtigkeit oder ein Loch im fluidführenden System, insbesondere des Abschnitts, durch welche(s) das im System geführte Fluid unerwünscht aus dem Inneren des Systems zu einem Äußeren des Systems austreten kann. Das Auftreten von Leckagen kann beliebige Ursachen haben; diese Ursachen sind an sich für die vorliegende Erfindung unerheblich.

Der Begriff "Absperreinrichtung", wie er hierin verwendet wird, bezeichnet eine am oder im System angeordnete Einrichtung, welche zum Verringern oder Unterbrechen bzw. Verhindern einer Strömung oder eines Flusses des medizinischen Fluids durch wenigstens einen Abschnitt des Systems geeignet und/oder vorgesehen ist.

In bestimmten Ausführungsformen der vorliegenden Erfindung ist die Absperreinrichtung vorgesehen zum Unterbrechen eines Ausströmens des Fluids aus dem Abschnitt in einen stromabwärts der Absperreinrichtung gelegenen Bereich.

Der Begriff "stromabwärts" ist in bestimmten erfindungsgemäßen Ausführungsformen als eine Strömungsrichtung innerhalb des Abschnitts zu verstehen, welche beim Durchführen des hierin beschrieben Verfahrens weg von der Fördereinrichtung führt.

Die Absperreinrichtung kann, ohne hierauf beschränkt zu sein, eine Klemme, wie eine arterielle oder eine venöse Klemme eines extrakorporalen Blutkreislaufs, eine Drossel, ein Ventil, ein Absperrhahn und dergleichen sein oder eines oder mehrere derartige Elemente aufweisen. Sie kann einteilig oder mehrteilig sein. Die Absperrwirkung der Absperreinrichtung kann sich aus dem Zusammenspiel verschiedener, also mehrerer Absperrkomponenten, oder aber allein einer Absperrkomponente, ergeben.

Der Begriff "Fördereinrichtung", wie er hierin verwendet wird, bezeichnet in bestimmten erfindungsgemäßen Ausführungsformen eine Einrichtung, welche zum Fördern des medizinischen Fluids in einem Inneren des Systems bzw. eines Abschnitts desselben oder durch das Innere oder den Abschnitt hindurch oder hieran entlang geeignet und/oder vorgesehen ist. Das Fördern des medizinischen Fluids kann mittelbar oder unmittelbar erfolgen.

Die konkrete Ausgestaltung der Fördereinrichtung ist erfindungsgemäß nicht beschränkt. Nicht einschränkende Beispiele schließen nicht-okkludierende Pumpen, wie eine Zentrifugalpumpe und dergleichen, ein.

Der Begriff "Förderanstrengung", wie er hierin in bestimmten Ausführungsformen der vorliegenden Erfindung verwendet wird, bezeichnet in bestimmten erfindungsgemäßen Ausführungsformen eine von der Fördereinrichtung zum Fördern des medizinischen Fluids verrichtete Leistung oder Arbeit. Dies kann z. B. mittels Spannungs- und/oder Strommessung am Eingang der Fördereinrichtung gemessen werden.

Die Förderanstrengung entspricht in einigen erfindungsgemäßen Ausführungsformen einer Förderleistung (z. B. in Milliliter pro Minute, ml/min), welche bei geöffneter Absperreinrichtung mittels der Fördereinrichtung im Abschnitt gefördert werden würde.

Die Förderanstrengung entspricht in manchen erfindungsgemäßen Ausführungsformen einer im Gebrauch der Fördereinrichtung veränderlichen Charakteristik der Einrichtung. Hierzu zählt eine eingestellte oder angestrebte oder ausgeführte Anzahl an Umdrehungen der Fördereinrichtung pro Minute.

Eine Förderanstrengung bezeichnet in bestimmten Ausführungsformen der vorliegenden Erfindung einen Zustand, in welchem sich die Fördereinrichtung während ihres Förderns des medizinischen Fluids befindet, insbesondere einen Zustand, auf welchen die Fördereinrichtung zum Fördern des medizinischen Fluids eingestellt wurde, z. B. durch Einstellen bzw. Vorgeben bestimmter Parameter, wie eines Förderdrucks, einer Förderleistung, einer Förderdrehzahl und dergleichen.

Das "Verändern einer Förderanstrengung", zum Beispiel das Verändern der ersten Förderanstrengung zu einer zweiten Förderanstrengung, kann durch Ändern wenigstens eines der für einen Förderzustand eingestellten bzw. einstellbaren Parameter, wie beispielsweise durch Ändern der Drehzahl, erreicht werden.

Dabei kann die von der Fördereinrichtung erbrachte - erste und/oder zweite - Förderanstrengung konstant oder nicht konstant sein. Vorzugsweise ist die von der Fördereinrichtung erbrachte erste und/oder die zweite Förderanstrengung im Wesentlichen oder vollständig konstant.

Die Begriffe erster und zweiter "Förderzustand" beschreiben in bestimmten erfindungsgemäßen Ausführungsformen den sich in Bezug auf einen sich im Abschnitt einstellenden ersten und zweiten Förderstrom stromabwärts der Absperreinrichtung als Ergebnis sowohl der Förderanstrengung der Fördereinrichtung als auch der Absperrwirkung der Absperreinrichtung einstellenden Zustand.

Der erste Förderzustand und der zweite Förderzustand können gleich oder voneinander verschieden sein. Wenigstens einer der beiden Förderzustände kann Null sein.

So kann ein erster Förderzustand Null sein, z. B. ausgedrückt durch einen Fluss von 0 ml/min, gemessen oder wenigstens messbar stromabwärts der Absperreinrichtung. Dies kann sich aufgrund der vollständigen Absperrung eines Stroms über die Absperreinrichtung hinweg ergeben. Ebenso kann ein zweiter Förderzustand Null sein, was sich z. B. aus einem vollständigen Anhalten der Fördereinrichtung ergeben kann. Allerdings ist die vorliegende Erfindung für den Fachmann erkennbar nicht auf Messungen oder Untersuchungen bei vollständiger Absperrung mittels der Absperreinrichtung oder vollständiges Unterbrechen des Förderstroms durch Anhalten der Fördereinrichtung beschränkt oder nur auf diese Weise ausführbar. Es ist vielmehr auch möglich, die Vorteile des erfindungsgemäßen Verfahrens zu erzielen bei nur teilweiser Absperrung der Absperreinrichtung und entsprechend nur teilweiser Drosselung der Fördereinrichtung. Auch diese Ausführungsformen sind von der vorliegenden Erfindung umfasst. Dies kommt zum Ausdruck durch die Verwendung des Begriffs "Förderzustand" wie oben dargelegt.

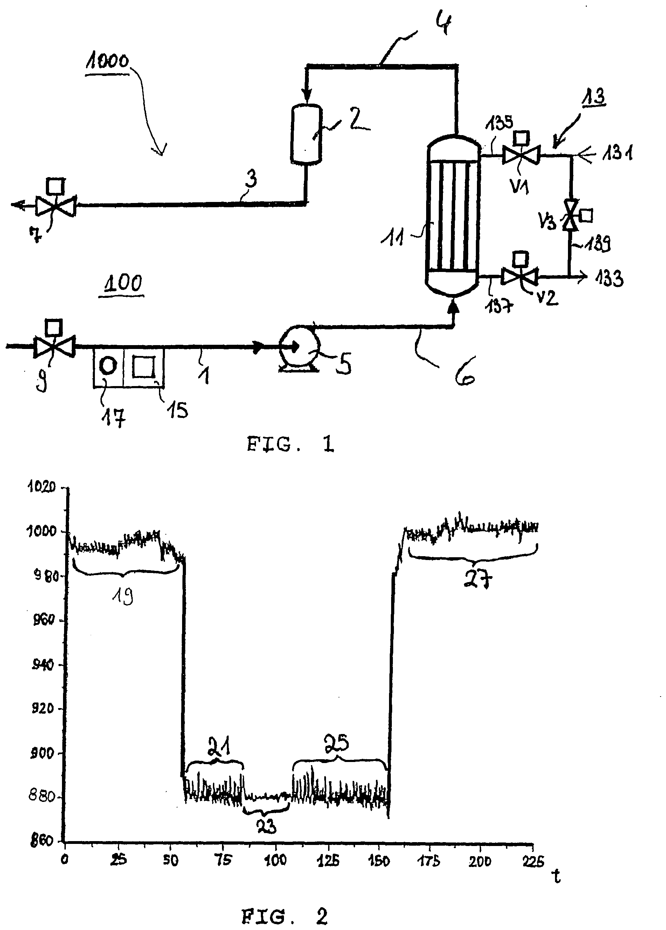

Von der Erfindung ist in bestimmten Ausführungsformen umfasst, dass zunächst ein erster Förderzustand betrachtet wird, anschließend der zweite Förderzustand. Allerdings ist die Erfindung hierauf nicht beschränkt. So ist die Reihenfolge der Untersuchung oder Messung beliebig, wie dies auch in den Fig. 2 bis 5 zu sehen ist. Beispielsweise kann in manchen Ausführungsformen zunächst die Fördereinrichtung angehalten oder gedrosselt werden und erst im Anschluss hieran die Absperreinrichtung gesperrt oder gedrosselt werden oder eben umgekehrt.

In bestimmten Ausführungsformen der vorliegenden Erfindung ist oder umfasst die emittierte Strahlung - wobei Strahlung hier als Beispiel zu verstehen ist für ein Signal wie hierin verwendet - elektromagnetische Strahlung wie beispielsweise sichtbares Licht.

In bestimmten Ausführungsformen der vorliegenden Erfindung ist oder umfasst die emittierte Strahlung Infrarotstrahlung, z. B. aus einer schmalbandigen Infrarotlichtquelle. Eine Spitzenwellenlänge der Infrarotstrahlung liegt vorzugsweise bei annähernd oder genau 805 nm.

Der Begriff "Strahlungsempfänger", wie er hierin verwendet wird, bezeichnet in bestimmten Ausführungsformen der vorliegenden Erfindung eine Einrichtung bzw. einen Sensor, welche(r) zum Empfangen und/oder Detektieren der aus dem Abschnitt des Systems emittierten Strahlung geeignet und/oder vorgesehen und/oder ausgestaltet ist.

Nicht einschränkende Beispiele für Strahlungsempfänger schließen optische Detektoren wie eine Fotodiode, einen Fotowiderstand oder einen Fototransistor und dergleichen ein.

Der Strahlungsempfänger kann wie die Strahlungsquelle einstückig oder mehrteilig ausgestaltet sein und/oder mittels einer Komponente zum Empfangen und/oder Emittieren von Strahlung ausgestaltet sein, oder durch mehrere. In manchen Ausführungsformen der vorliegenden Erfindung ist der Strahlungsempfänger als eigenes und/oder eigenständiges Bauteil vorgesehen und ausgestaltet. In manchen Ausführungsformen der vorliegenden Erfindung ist der Strahlungsempfänger in einer gemeinsamen körperlichen Anordnung, wie einem gemeinsamen Gehäuse, mit der Strahlungsquelle vorgesehen.

Der Begriff "Signalempfangseinrichtung" , wie er hierin verwendet wird, geht inhaltlich über den oben erläuterten Begriff "Strahlungsempfänger" hinaus. Eine Signalempfangseinrichtung kann ein Strahlungsempfänger sein, ist aber nicht auf das Empfangen von Strahlung beschränkt. Statt - oder zusätzlich zu - Strahlung kann ein anders Signal, z. B. ein Ultraschallsignal, empfangen werden. Dieselbe Beziehung trifft auf die Begriffe "Strahlungsquelle" und "Signalsendeeinrichtung" zu.

Der Begriff "Empfangen" eines Anteils des emittierten Signals, z. B. der emittierten Strahlung, wie er hierin verwendet wird, bezeichnet in bestimmten Ausführungsformen der vorliegenden Erfindung ein gezieltes Empfangen oder Detektieren des aus dem Abschnitt des Systems emittierten Signals, z. B. der emittierten Strahlung.

Beim "Anteil der emittierten Strahlung" kann es sich um einen den Abschnitt - z. B. durch Reflexion, Transmission, Streuung usw. - verlassenden oder um einen in den Abschnitt eingedrungenen und darin gemessenen Anteil eines Signals, z. B. der Strahlung, handeln.

Der Begriff "Anteil", wie er hierin verwendet wird, bezeichnet in bestimmten Ausführungsformen der vorliegenden Erfindung einen Teil, z. B. einen Bruchteil oder eine Teilmenge, den das empfangene Signal, z. B. die empfangene Strahlung, gemessen am ursprünglich emittierten Signal ausmacht.

Der Anteil des emittierten Signals, z. B. der Strahlung, der nach Emission wieder empfangen wird, ist in manchen erfindungsgemäßen Ausführungsformen ein Bruchteil einer Intensität (z. B. gemessen als Amplitude eines Signals, als Counts, als Counts pro Zeiteinheit, als elektrische Spannung nach entsprechender Wandelung, elektrischer Strom, Frequenz, usw.).

Counts können dabei beispielsweise, ohne hierauf beschränkt zu sein, wie folgt erhalten werden: Bei Verwenden einer Signalempfangseinrichtung, welche als ein Lichtempfänger ausgestaltet ist, der als ein Light-To-Frequency-Konverter wirkt, gibt der verwendete Sensor eine der empfangenen Lichtintensität proportionale Frequenz aus. Zur Auswertung werden z. B. die Flanken des Signals für eine bestimmte Zeiteinheit gezählt, jede Flanke wird dabei als Count gewertet.

In manchen Ausführungsformen der vorliegenden Erfindung ist dieser das System bzw. dessen Abschnitt verlassende Anteil des emittierten Signals oder der emittierten Strahlung ausschließlich oder auch ein reflektiertes Signal. In einigen Ausführungsformen der vorliegenden Erfindung ist der das System bzw. dessen Abschnitt verlassende Anteil des emittierten Signals oder der emittierten Strahlung ausschließlich oder auch ein transmittiertes Signal. In bestimmten Ausführungsformen der vorliegenden Erfindung ist der das System bzw. dessen Abschnitt verlassende Anteil des emittierten Signals, z. B. der emittierten Strahlung, ein ausschließlich oder auch gestreutes, z. B. seitwärts gestreutes, Signal, z. B. Strahlung.

Zum Treffen einer Aussage darüber, ob eine Leckage vorliegt, anhand einer Auswertung des ersten Anteils in Bezug auf den zweiten Anteil, ist in bestimmten erfindungsgemäßen Ausführungsformen ein Vergleichen des ersten mit dem zweiten Anteil, bzw. der Höhen oder Ausprägungen, vorgesehen.

Das Vergleichen des ersten Anteils mit dem zweiten Anteil erfolgt in bestimmten Ausführungsformen der vorliegenden Erfindung durch Vergleichen eines ersten Mittelwerts eines über einen bestimmten ersten Zeitraum als ersten Anteil empfangenen Signals mit einem zweiten Mittelwert eines über einen bestimmten zweiten Zeitraum als zweiten Anteil empfangenen Signals.

Der Vergleich erfolgt in manchen Ausführungsformen der vorliegenden Erfindung durch Subtraktion des ersten Anteils oder seines Mittelwerts vom zweiten Anteil oder seines Mittelwerts unter Erhalten einer Differenz oder eines Differenzwerts.

In manchen Ausführungsformen erfolgt der Vergleich durch einen Vergleich von Signal- oder Strahlungsspektren des ersten und zweiten Anteils des empfangenen Signals oder der empfangenen Strahlung. Zum Beispiel können die Absolutwerte von Signal- oder Strahlungsmaxima und/oder Signal- oder Strahlungsminima der aufgezeichneten Signal- oder Strahlungsspektren verglichen werden.

Der Vergleich erfolgt in manchen Ausführungsformen der vorliegenden Erfindung durch Bilden eines Verhältnisses zwischen dem ersten Anteil oder seinem ersten Mittelwert und dem zweiten Anteil oder seinem zweiten Mittelwert.

In bestimmten Ausführungsformen der vorliegenden Erfindung umfasst das Treffen einer Aussage darüber, ob eine Leckage vorliegt einen Vergleich mit einem Schwellenwert oder besteht hieraus. Dabei kann eine Differenz, ein Verhältnis oder ein auf andere Weise erhaltener Wert mit dem Schwellenwert verglichen werden. Die Differenz oder das Verhältnis können insbesondere wie oben beschrieben ermittelt werden.

Bei dem Schwellenwert kann es sich insbesondere um einen vorbestimmten Schwellen- oder Referenzwert, wie beispielsweise einen Schwellenwert, der in einem System bzw. Abschnitt ohne Leckage erfasst, berechnet, geschätzt oder dergleichen wurde, handeln.

In bestimmten Ausführungsformen der vorliegenden Erfindung ist der erste und/oder der zweite Anteil eine prozentuale Signal- oder Strahlungsintensität (I) oder spiegelt diese wider.

In bestimmten Ausführungsformen der vorliegenden Erfindung wird die Signalempfangseinrichtung, insbesondere wenn als Strahlungsempfänger ausgestaltet, zum Erkennen einer optischen Dichte oder einer Änderung hiervon eingesetzt. Letzteres mag zum Erkennen einer Leckage dienen, nicht aber zwingend erforderlich sein.

In solchen Ausführungsformen kann es beispielsweise vorteilhaft möglich sein, zwischen dem Vorhandensein von Blut oder Wasser in einem extrakorporalen Blutkreislauf zu unterscheiden.

Zur Durchführung des erfindungsgemäßen Verfahrens wird in bestimmten Ausführungsformen des erfindungsgemäßen Verfahrens ein Fluidfluss des medizinischen Fluids durch den Abschnitt des Systems im ersten Förderzustand mittels der Absperreinrichtung angehalten. Die Strömung kann gestoppt, d. h. auf Null eingestellt werden. In anderen erfindungsgemäßen Ausführungsformen wird der Fluidfluss mittels der Absperreinrichtung nur geeignet gedrosselt, nicht aber vollständig unterbunden.

Die Fördereinrichtung kann dabei weiter fördern oder auch nicht.

In bestimmten Ausführungsformen des erfindungsgemäßen Verfahrens ist vorgesehen, die Fördereinrichtung im zweiten Förderzustand anzuhalten. In anderen erfindungsgemäßen Ausführungsformen wird die Fördereinrichtung nur gedrosselt, nicht aber vollständig angehalten.

Sollte sich aus der Durchführung des erfindungsgemäßen Verfahrens ergeben oder vermutet werden, dass eine Leckage im fluidführenden System vorliegt, ist in bestimmten Ausführungsformen der vorliegenden Erfindung vorgesehen, einen Alarm auszugeben. Der Alarm kann je nach Wunsch und/oder Bedarf bzw. Erfordernis ein optischer, akustischer oder in jedweder anderen Art geeigneter Alarm sowie eine Kombination verschiedener Alarme sein.

Alle, einige oder manche Schritte eines erfindungsgemäßen Verfahrens, wie es beispielhaft und nicht einschränkend anhand der beigefügten Zeichnung beschrieben ist, können automatisch und/oder automatisiert durchgeführt werden. Für jeden der im Zusammenhang mit dem erfindungsgemäßen Verfahren beschriebenen Verfahrensschritte können die erfindungsgemäßen Vorrichtungen entsprechende Einrichtungen zu deren Umsetzung aufweisen.

In bestimmten Ausführungsformen der vorliegenden Erfindung weist das erfindungsgemäße System wenigstens eine Behandlungskassette mit wenigstens dem ein medizinisches Fluid führenden Abschnitt, eine Fördereinrichtung zum Fördern des Fluids durch den Abschnitt sowie eine Absperreinrichtung zum Unterbrechen oder Verringern des Fluidstroms durch den Abschnitt auf.

Der Begriff "Behandlungskassette", wie er hierin verwendet wird, bezeichnet eine Funktionseinrichtung, welche zum Durchführen einer medizinischen Behandlung, z. B. einer extrakorporalen Behandlung von Blut, vorgesehen und/oder eingesetzt ist oder wird.

Beispiele für Behandlungskassetten schließen eine Blutkassette, z. B. in Form eines Gussteils oder Spritzgussteils, unabhängig davon, ob diese als Einwegartikel oder Disposable ausgestaltet ist oder nicht, ein.

In bestimmten Ausführungsformen des erfindungsgemäßen Systems ist zumindest die Fördereinrichtung Teil der Behandlungskassette.

Das erfindungsgemäße System weist in bestimmten Ausführungsformen eine Strahlungsquelle zum Emittieren von Strahlung als Signalsendeeinrichtung auf.

Die Strahlungsquelle ist in bestimmten Ausführungsformen ausgestaltet, um elektromagnetische Strahlung, insbesondere Infrarotlicht, zu emittieren.

Die Signalsendeeinrichtung ist in manchen Ausführungsformen ausgestaltet, um Ultraschallwellen zu emittieren.

In bestimmten erfindungsgemäßen Ausführungsformen weist das System eine Signalempfangseinrichtung, konfiguriert und/oder vorgesehen zum Empfangen eines Anteils des emittierten Signals und eine Steuereinrichtung zum Durchführen des erfindungsgemäßen Verfahrens auf.

In bestimmten Ausführungsformen ist die Signalempfangseinrichtung konfiguriert und/oder vorgesehen und/oder ausgestaltet, um reflektierte und/oder transmittierte und/oder gestreute Signale zu detektieren.

Die Signalempfangseinrichtung ist in bestimmten erfindungsgemäßen Ausführungsformen als Einrichtung zum Empfangen von Ultraschallwellen ausgestaltet.

Die Signalempfangseinrichtung ist in manchen erfindungsgemäßen Ausführungsformen als Einrichtung zum Empfangen von Strahlung ausgestaltet.

Das erfindungsgemäße System weist in bestimmten Ausführungsformen ferner wenigstens eine Vergleichseinrichtung auf zum Vergleichen des im ersten Förderzustand empfangenen ersten Anteils mit dem im zweiten Förderzustand empfangenen zweiten Anteil des emittierten Signals, z. B. der emittierten Strahlung und/oder der Ultraschallwellen.

In bestimmten Ausführungsformen des erfindungsgemäßen Systems ist ferner eine Entscheidungseinrichtung, konfiguriert und/oder vorgesehen zum Treffen einer Aussage darüber, ob eine Leckage vorliegt, anhand einer Auswertung des ersten Anteils in Bezug auf den zweiten Anteil im oder am System vorgesehen.

In bestimmten erfindungsgemäßen Ausführungsformen weist das System ferner wenigstens eine Alarmeinrichtung auf, die konfiguriert ist zum Ausgeben eines Alarms bei erkannter Leckage.

In einigen erfindungsgemäßen Ausführungsformen kommt keine Gaspumpe und/oder kein Durchflussmesser zum Messen von Gasströmung zum Einsatz oder diese sind nicht vorgesehen, und/oder es wird kein Gasdurchfluss gemessen.

In manchen Ausführungsformen wird bei der Durchführung des erfindungsgemäßen Verfahrens kein Unterdruck angelegt. Entsprechend sind Unterdruckeinrichtungen in bestimmten erfindungsgemäßen Ausführungsformen nicht vorgesehen und/oder werden bei der Durchführung des erfindungsgemäßen Verfahrens nicht verwendet.

In bestimmten erfindungsgemäßen Ausführungsformen wird kein absolutes Messergebnis erhoben.

In gewissen erfindungsgemäßen Ausführungsformen wird keine Fluss- oder Strömungsrate ermittelt.

Bestimmte erfindungsgemäße Ausführungsformen weisen einen oder mehrere der im Folgenden genannten Vorteile auf.

Die vorliegende Erfindung stellt ein Verfahren sowie Vorrichtungen bereit, mittels welchen in manchen erfindungsgemäßen Ausführungsformen vorteilhaft auf einfache Weise eine Detektion von Leckagen - gleich welcher Ursache - in fluidführenden Systemen möglich ist.

Da sich die Intensität des detektierten Signals, z. B. der detektierten Strahlung, des fließenden Bluts von der bei stillstehendem Blut unterscheidet, kann es in bestimmten Ausführungsformen der vorliegenden Erfindung beispielsweise auf vorteilhaft einfache Weise möglich sein, eine Änderung der Blutzellenverteilung im Abschnitt des Systems zu beobachten bzw. optisch zu erfassen und anhand der Änderung in einfacher Weise auf eine Leckage des blutführenden Systems zu schließen.

Druckhaltetests, welche üblicherweise zur Überprüfung der Dichtigkeit fluidführender Systeme mit okkludierenden Pumpen wie Rollenpumpen, Schlauchpumpen, Verdrängerpumpen usw. etabliert sind, sind bei Konstantdruckquellen, d. h. nicht-okkludierenden Pumpen wie Zentrifugalpumpen zur Detektion von Leckagen prinzipbedingt nicht möglich, da der Druck auch bei kleinen Leckagen konstant bleibt. Die vorliegende Erfindung bietet für solche Systeme mit nicht-okkludierenden Pumpen vorteilhaft eine einfache und wenig aufwendige Möglichkeit, Leckagen dennoch zu erfassen.

Die Verwendung eines optischen Sensors kann hierbei insbesondere von Vorteil sein, um eine hohe Genauigkeit auch bei kleinen Leckagen zu erreichen, welche mittels z. B. Flusssensoren nicht möglich ist.

Daneben ist der erfindungsgemäß eingesetzte optische Sensor ein vorteilhaft einfacher Sensor, was gleichzeitig dazu beitragen kann, den konstruktiven und/oder finanziellen Aufwand des Systems zu reduzieren.

Ferner kann es in bestimmten Ausführungsformen der vorliegenden Erfindung vorteilhaft möglich sein, weitere Messungen, wie beispielsweise eine Unterscheidung zwischen Blut und Wasser und/oder Luft oder Hämatokrit- bzw. Hämoglobinkonzentrationsmessungen und dergleichen mit ein-und demselben Sensor durchzuführen, der auch zum Durchführen des erfindungsgemäßen Verfahrens zum Einsatz kommen kann.

Im Folgenden wird die vorliegende Erfindung unter Bezugnahme auf die beigefügten Figuren rein exemplarisch beschrieben. In der Zeichnung bezeichnen identische Bezugszeichen gleiche oder identische Elemente. Es gilt:
- Fig. 1: zeigt schematisch einen Abschnitt eines Systems gemäß der vorliegenden Erfindung;
- Fig. 2: zeigt den Verlauf eines optischen Signals bei einem Versuchsaufbau ohne Leckage;
- Fig. 3: zeigt den Verlauf eines optischen Signals bei einer Leckage, erzeugt durch eine 0,4 x 19 mm-Kanüle;
- Fig. 4: zeigt den Verlauf eines optischen Signals bei einer weiteren Leckage, erzeugt durch eine 0,6 x 25 mm-Kanüle; und
- Fig. 5: zeigt den Verlauf eines optischen Signals bei einer wiederum weiteren Leckage, erzeugt durch eine 0,6 x 25 mm-Kanüle bei einer Transmissionsmessung.

Zur Verdeutlichung des erfindungsgemäßen Verfahrens wurden die Zeitintervalle der Messungen in den folgenden beispielhaften Ausführungsformen sehr lang gewählt, um zum Zwecke der Beschreibung der vorliegenden Erfindung eine möglichst anschauliche und deutliche Darstellung zu erhalten. Natürlich kann die Dauer für die Durchführung des erfindungsgemäßen Verfahrens (deutlich) kürzer sein.

Die folgenden Beispiele sind zudem anhand von Strahlung als Signal ausgeführt. Für den Fachmann ist erkennbar, dass die vorliegende Erfindung nicht auf die Verwendung von Strahlung als Signal beschränkt ist. Anstelle von Strahlung kann vielmehr auch eine andere Signalart verwendet werden, wie dies oben ausgeführt ist.

**Fig. 1** zeigt schematisch einen Abschnitt eines Systems 1000 gemäß der vorliegenden Erfindung.

Wie in Fig. 1 gezeigt, ist das System 1000 als extrakorporaler Blutkreislauf ausgestaltet. Das System 1000 kann ein (z. B. Kunststoff-) Schlauchset aufweisen oder sein.

Das System weist eine arterielle Blutleitung 1 sowie eine venöse Blutleitung 3 mit einer venösen Kammer 31 auf.

Zum Fördern des Bluts in einem Leitungsinneren des extrakorporalen Blutkreislaufs ist eine Fördereinrichtung 5, z. B. in Form einer Zentrifugalpumpe oder jeder anderen Ausgestaltung, welche z. B. überströmbar oder nicht okkludierend ist, vorgesehen.

Der venöse Schenkel des extrakorporalen Blutkreislaufs weist eine Tropfkammer 2 auf. Die Tropfkammer 2 weist eine nicht dargestellte, herkömmliche Entlüftungseinrichtung auf. Die Entlüftungseinrichtung ist in manchen erfindungsgemäßen Ausführungsformen ein Luftablassventil. In bestimmten erfindungsgemäßen Ausführungsformen weist die Entlüftungseinrichtung eine Membran, vorzugsweise eine hydrophobe Membran, auf.

In Fig. 1 ist die Richtung des extrakorporalen Flusses des Bluts im Leitungsinneren des extrakorporalen Blutkreislaufs während einer Blutbehandlung durch die Richtung der ausgefüllten Pfeilspitzen angedeutet.

Das System 1000 weist eine venöse Klemme auf, welche im Rahmen der erfindungsgemäßen Ausführungsform der Fig. 1 beispielhaft als Absperreinrichtung 7 dient.

Das System 1000 weist ferner eine arterielle Klemme 9 auf. Die arterielle Klemme 9 kann, muss aber nicht, zusätzlich oder alternativ zur Absperreinrichtung 7, als Absperreinrichtung im Rahmen der vorliegenden Erfindung eingesetzt werden.

Zur extrakorporalen Blutbehandlung ist eine Blutbehandlungseinrichtung 11 im extrakorporalen Blutkreislauf angeordnet bzw. steht mit diesem in Fluidkontakt.

Die Blutbehandlungseinrichtung 11 steht im Beispiel der Fig. 1 mittels einem venösen Leitungsabschnitt 4 mit der Tropfkammer 2 in Verbindung. Sie steht ferner mittels einem arteriellen Leitungsabschnitt 6 mit der Fördereinrichtung 5 in Verbindung.

Beispiele für die Blutbehandlungseinrichtung 11 umfassen einen Blutfilter zum Reinigen von Patientenblut im Rahmen einer Hämodialyse- und/oder Hämofiltrationsbehandlung und dergleichen, sind jedoch nicht hierauf beschränkt. Die Blutbehandlungseinrichtung 11 kann als Einwegprodukt oder Disposable-Einrichtung ausgestaltet sein.

Die Blutbehandlungseinrichtung 11 steht ferner mit einem Dialysatkreislauf 13 in Fluidkontakt.

Der Dialysatkreislauf 13 weist einen Dialysatzulauf 131 sowie einen Dialysatablauf 133 auf bzw. ist zum Zwecke des Zu- bzw. Abführens von Dialysat mit geeigneten und/oder vorgesehenen Einrichtungen verbunden. Der Dialysatkreislauf 13 weist eine in Fig. 1 nicht gezeigte, herkömmliche Dialysatpumpe auf zum Fördern von Dialysat innerhalb des Dialysatkreislaufs 13.

In der zur Blutbehandlungseinrichtung 11 vom Dialysatzulauf 131 führenden Zuleitung 135 ist ein erstes Ventil V1 im Dialysatkreislauf 13 angeordnet.

In der von der Blutbehandlungseinrichtung 11 zum Dialysatablauf 133 führenden Ableitung 137 ist ein zweites Ventil V2 angeordnet.

Die Zuleitung 135 und die Ableitung 137 des Dialysatkreislaufs 13 stehen über eine Verbindungsleitung 139, welche optional ein Bypassventil V3 aufweist, in Fluidverbindung.

Wie in Fig. 1 gezeigt, ist in der arteriellen Blutleitung 1 des extrakorporalen Blutkreislaufs ein Strahlungsempfänger 15 angeordnet.

Der Strahlungsempfänger 15 kann ein optischer Detektor sein. Der Strahlungsempfänger 15 kann z. B. dazu vorgesehen und/oder ausgelegt und/oder konfiguriert sein, um Änderungen in der Lichtintensität zwischen emittierter und empfangener Strahlung, verursacht durch das Vorhandensein und ggf. die Bewegung der roten Blutkörperchen des extrakorporal fließenden Bluts zu registrieren.

Der Strahlungsempfänger 15 kann, wie hier in Fig. 1 gezeigt, einstückig, d. h. in einem gemeinsamen Körper, mit einer Strahlungsquelle 17, z. B. einer Infrarotquelle, ausgestaltet sein.

In anderen Ausführungsformen, hier nicht gezeigt, können Strahlungsempfänger 15 und Strahlungsquelle 17 jedoch auch körperlich getrennt voneinander ausgestaltet und/oder räumlich getrennt voneinander angeordnet sein.

Um eine potentielle Leckage im Abschnitt 100 des Systems 1000 zu detektieren, werden in einer exemplarischen Ausführungsform des erfindungsgemäßen Verfahrens zunächst die Absperreinrichtung 7 sowie die Ventile V1 und V2 im Dialysatkreislauf 13 geschlossen.

Dies kann in bestimmten Ausführungsformen des erfindungsgemäßen Verfahrens erfolgen, ohne eine bereits vorliegende, konstante Drehzahl der Fördereinrichtung 5 zu ändern oder ohne eine andere konstante Drehzahl als eine jener konstanten Drehzahlen einzustellen, die während der Patientenbehandlung ohnehin oder regelmäßig zum Einsatz kommen. In anderen Ausführungsformen kann sich die Drehzahl der Fördereinrichtung 5 jedoch durchaus auch ändern oder zum Durchführen des hier beschriebenen Verfahrens geändert werden.

Das Ventil V3 - falls vorhanden - wird anschließend geöffnet.

Auf diese Weise wird eine statische Druckdifferenz über der Fördereinrichtung 5 aufgebaut.

Während des hiermit erzielten oder eingestellten ersten Förderzustands, welcher bei Abwesenheit eines Lecks/einer Leckage stromabwärts der Fördereinrichtung 5 0 ml/min (in Worten: Null) beträgt oder betragen kann, empfängt der Strahlungsempfänger 15 jenen Anteil der emittierten Strahlung, welcher durch das Blut reflektiert wird. Dieser Anteil ist als der erste Anteil zu verstehen.

Um aus Referenzgründen einen weiteren, als zweiten bezeichneten Anteil des von der Strahlungsquelle 17 emittierten Lichts in einem zweiten Förderzustand der Fördereinrichtung 5 zu erfassen, wird die Fördereinrichtung 5 für eine bestimmte Zeit gestoppt. Der zweite Förderzustand entspricht daher zuverlässig einem Flussstillstand des Bluts innerhalb des betrachteten Abschnitts des extrakorporalen Blutkreislaufs.

Das bei Flussstillstand aufgezeichnete Signal (zweiter Anteil) des zweiten Förderzustands wird mit dem Signal bei drehender Fördereinrichtung 5 (erster Anteil) des ersten Förderzustands verglichen.

Nach einer unbestimmten oder vorbestimmten, jedenfalls aber ausreichenden Zeit wird die Fördereinrichtung 5 wieder gestartet.

Unterscheidet sich der erste Anteil vom zweiten Anteil, z. B. bei Betrachtung von deren Mittelwerten, des unverändert emittierten optischen Signals, d. h. zwischen kontinuierlich drehender Fördereinrichtung 5 und stehender Fördereinrichtung 5, so kann auf das Vorhandensein eines Blutlecks im extrakorporalen Blutkreislauf, z. B. eine Undichtigkeit in einer der beiden Patientenleitungen 1, 3 eines Schlauchsets und/oder eine Undichtigkeit einer Konnektierung zur Blutbehandlungseinrichtung 11, geschlossen werden. Ein entsprechendes Alarmsignal kann ausgegeben werden.

Wird keine Änderung des Signals oder seines Mittelwerts oder eine andere mathematische Auswertung hiervon detektiert, dann ist der Leckagetest bestanden.

Möglich und erfindungsgemäß angedacht ist auch folgendes Vorgehen: Bei geschlossenem Ventil V2 wird mittels der (nicht dargestellten) Dialysatpumpe oder einer anderen entsprechend geschalteten Pumpe eine Flüssigkeit, z. B. Dialysat, über die Membran der Blutbehandlungseinrichtung 11 auf die Blutseite gefördert. Dort verteilt sich die auf die Blutseite übergetretene Flüssigkeit sowohl auf den venösen Leitungsabschnitt 4 als auch auf den arteriellen Leitungsabschnitt 6. Über die Tropfkammer 2 und die, insbesondere im Stillstand, überspülbare Fördereinrichtung 5 jeweils hindurch oder hinweg können auch die arterielle Blutleitung 1 und die venöse Blutleitung 3 gespült werden. Auf diese Weise kann das gesamte System 1000 mit Flüssigkeit gefüllt werden. Die arterielle Blutleitung 1 und die venöse Blutleitung 3 können dabei direkt oder über einen Adapter oder dergleichen kurzgeschlossen oder miteinander verbunden werden. Alternativ werden die arterielle Blutleitung 1 und die venöse Blutleitung 3 nicht miteinander verbunden. Die sie durchströmende Flüssigkeit kann verworfen werden. Im Anschluss an das hier beschriebene Vorgehen kann die Fördereinrichtung 5 betrieben werden, um ggf. im System 1000 vorliegende Luft mittels der Tropfkammer 2 oder ihrer Entlüftungseinrichtung aus dem System 1000 zu entfernen.

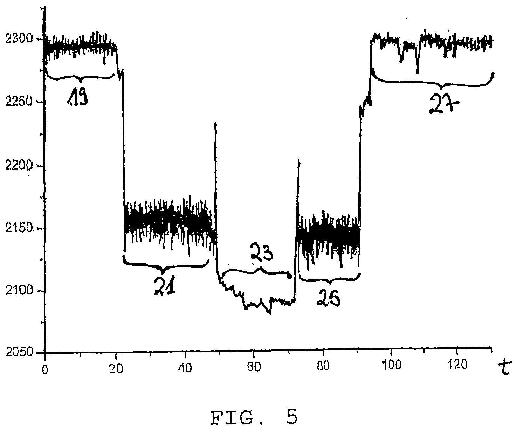

In den folgenden Fig. 2 bis 4 ist jeweils der Verlauf des optischen Signals der reflektierten Strahlung als eine der Lichtintensität proportionale Zahl über der Zeit t dargestellt. In Fig. 5 ist hingegen der Verlauf des optischen Signals der transmittierten Strahlung gemessen als eine der Lichtintensität proportionale Zahl über der Zeit t (z. B. in Sekunden oder einer anderen Einheit) dargestellt.

Zum leichteren Verständnis werden in den folgenden Versuchsbeschreibungen zu den Fig. 2 bis 5 jeweils die Bezugszeichen der im Abschnitt 100 des erfindungsgemäßen Systems 1000 gezeigten Komponenten verwendet, obgleich diese Komponenten zum Teil in den im Folgenden beschriebenen Figuren nicht gezeigt sind.

**Fig. 2** zeigt den Verlauf des optischen Signals bei einem Versuchsaufbau ohne Leckage.

Bei diesem Versuchsaufbau, welcher zum Ergebnis der Fig. 2 geführt hat, wurde die Fördereinrichtung 5 zunächst mit einer kontinuierlichen Drehzahl von 4500 U/min betrieben. Das Schlauchset wies kein Leck auf.

Die Absperreinrichtung 7 war zunächst geöffnet (Bereich 19). Ohne Änderung der Drehzahl der Fördereinrichtung 5 wurde sie nach etwas mehr als 50 Zeiteinheiten geschlossen (Bereich 21; entspricht dem ersten Förderzustand). Der Mittelwert des ersten Anteils der empfangenen Strahlung liegt in Fig. 2 leicht erkennbar bei etwa 880 Maß- oder Dimensionseinheiten.

Anschließend wurde die Fördereinrichtung 5 angehalten (Bereich 23; entspricht dem zweiten Förderzustand) und etwas später, z. B. nach 25 Zeiteinheiten wie in Fig. 2 gezeigt, wieder gestartet (Bereich 25). Der Mittelwert des zweiten Anteils der empfangenen Strahlung liegt ebenfalls bei etwa 880 Maß- oder Dimensionseinheiten.

Eine Differenz zwischen den beiden Mittelwerten (erster Anteil und zweiter Anteil) ergibt somit etwa ± 0 Maß- oder Dimensionseinheiten.

Ein Vergleich der Differenz mit einem hier nicht angegebenen Schwellenwert würde somit - mangels einer Differenz - zu dem Ergebnis führen, dass eine Leckage nicht nachweisbar ist.

Gegen Ende des Versuchs wird die Absperreinrichtung 7 wieder geöffnet (Bereich 27).

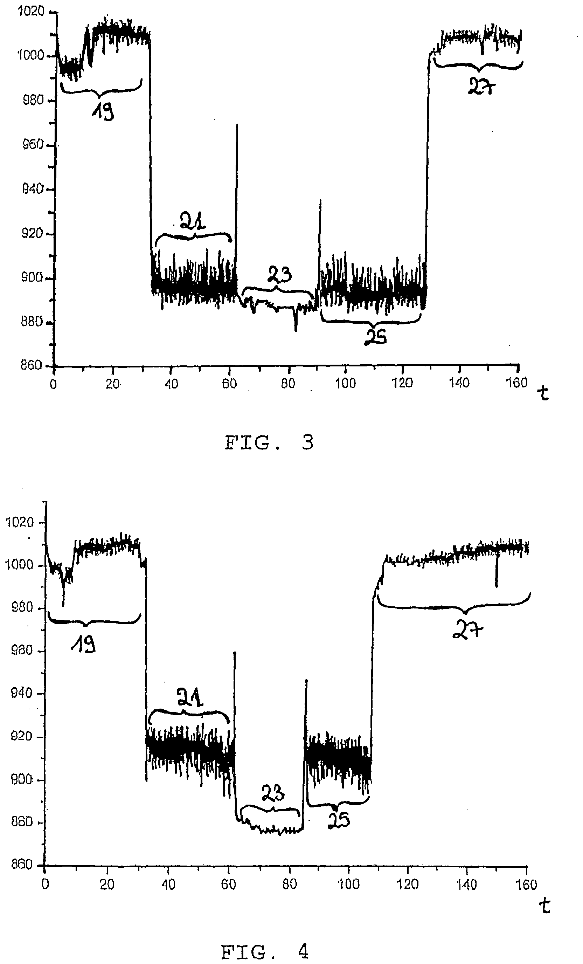

Der anhand des Verlaufs der Kurve aus Fig. 2 beschriebene Versuch wurde mit gezielt gesetzten Leckagen im Schlauchsatz wiederholt: In den anhand von Fig. 3 bis 5 gezeigten Versuchsdurchführungen wurde jeweils eine offene Spritze (ohne Kolben) in ein Septum gestochen. Auf diese Weise konnte über die Wahl der Kanüle ein Leck definierter Größe geschaffen werden.

**Fig. 3** zeigt den Verlauf eines optischen Signals bei einer Leckage aufgrund Punktion des Schlauchsatzes mittels einer 0,4 x 19 mm-Kanüle.

Wie aus Fig. 3 zu erkennen ist, lag der Mittelwert des optischen Signals bei zirkulierender oder fördernder Fördereinrichtung 5 und geschlossener Absperreinrichtung 7 (Bereich 21; Mittelwert liegt bei etwa 889 Dimensionseinheiten), anders als beim Versuch ohne Leckage (Fig. 2), höher als bei einer stehenden Fördereinrichtung 5 (Bereich 23; Mittelwert liegt bei etwa 881 Dimensionseinheiten). Der Differenzwert betrug etwa 8 Dimensionseinheiten. Dies kann auf den aufgrund der Leckage möglichen sehr niedrigen Blutfluss zurückgeführt werden.

Bei den Maß- oder Dimensionseinheiten kann es sich z. B. um Counts handeln, die wie folgt erhalten werden: Die im Beispiel der hier beschriebenen Figuren verwendete Signalempfangseinrichtung ist ein Lichtempfänger, der als ein Light-To-Frequency-Konverter ausgestaltet ist. Der verwendete Sensor gibt also eine der empfangenen Lichtintensität proportionale Frequenz aus. Zur Auswertung werden z. B. die Flanken des Signals für eine bestimmte Zeiteinheit gezählt, jede Flanke wird dabei als Count gewertet.

Bei entsprechend festgelegtem Schwellenwert kann durch Vergleich der Differenz hiermit ein Leckalarm ausgegeben werden.

**Fig. 4** zeigt den Verlauf eines optischen Signals bei einer mittels einer 0,6 x 25 mm-Kanüle erzeugten Leckage bei einer Reflexionsmessung.

In Fig. 4 ist gut zu erkennen, dass der Signalverlauf bei zirkulierender Fördereinrichtung 5 und gesperrter Absperreinrichtung 7 deutlich höher als der Signalverlauf bei stehender Fördereinrichtung 5 ist.

Der in Fig. 4 zu sehende, gemessen am Verlauf der Fig. 3 höhere Signalunterschied kann der im Vergleich zur 0,4 x 19 mm-Kanüle aus Fig. 3 mit der in Fig. 4 mit der 0,6 x 25 mm-Kanüle erzeugten, größeren Leckage und dem damit zugelassenen größeren Fluss (trotz Sperrung) zugeschrieben werden.

**Fig. 5** zeigt den Verlauf eines optischen Signals bei einem Leck durch eine 0,6 x 25 mm-Kanüle bei einer Transmissionsmessung. Es gilt ansonsten das zu den Fig. 2 bis 4 Gesagte.

## Patentansprüche

1. Verfahren zum Detektieren von Leckagen eines ein medizinisches Fluid führenden Systems (1000) stromaufwärts einer Absperreinrichtung (7) des Systems (1000), wobei
- das System (1000) einen stromaufwärts der Absperreinrichtung (7) gelegenen, das medizinische Fluid führenden Abschnitt (100) aufweist;
- die Absperreinrichtung (7) zum Unterbrechen oder Verringern eines Ausströmens des Fluids aus dem Abschnitt (100) in einen stromabwärts der Absperreinrichtung (7) gelegenen Bereich vorgesehen ist;
- das System (1000) ferner wenigstens eine Fördereinrichtung (5) zum Fördern des Fluids durch den Abschnitt (100) hindurch, aufweist;
wobei das Verfahren die Schritte umfasst:
- Unterbrechen oder Verringern des Fluidstroms durch den Abschnitt (100) hindurch oder aus diesem heraus während einer ersten Förderanstrengung der Fördereinrichtung (5) mittels Einstellen der Absperreinrichtung (7) zum Erzielen eines ersten Förderzustands im Abschnitt (100);
- Erzielen eines zweiten Förderzustands im Abschnitt (100) durch Verändern der ersten Förderanstrengung zu einer zweiten Förderanstrengung der Fördereinrichtung (5);
**dadurch gekennzeichnet, dass** das Verfahren die weiteren Schritte umfasst:
- Emittieren wenigstens eines Signals mittels einer Signalsendeeinrichtung in den Abschnitt (100) des Systems (1000) hinein sowohl im ersten als auch im zweiten Förderzustand;
- Empfangen eines den Abschnitt (100) verlassenden Anteils des emittierten Signals mittels einer Signalempfangseinrichtung im ersten Förderzustand als einen ersten Anteil;
- Empfangen eines den Abschnitt (100) verlassenden Anteils des emittierten Signals mittels der Signalempfangseinrichtung im zweiten Förderzustand als einen zweiten Anteil; und
- Treffen einer Aussage darüber, ob eine Leckage vorliegt, anhand einer Auswertung des ersten Anteils in Bezug auf den zweiten Anteil, welche einen Vergleich des ersten Anteils mit dem zweiten Anteil und/oder einer zwischen erstem und zweitem Anteil gebildeten Differenz mit einem Schwellenwert umfasst oder hieraus besteht.

2. Verfahren nach Anspruch 1, wobei das emittierte Signal Ultraschallwellen und/oder Strahlung umfasst oder hieraus besteht, wobei die Signalsendeeinrichtung Ultraschallwellen emittiert und/oder eine Strahlungsquelle (17) zum Emittieren von Strahlung ist, und wobei die Signalempfangseinrichtung Ultraschallwellen detektiert und/oder ein Strahlungsempfänger (15) zum Empfangen oder Detektieren von Strahlung ist.

3. Verfahren nach Anspruch 2, wobei die emittierte Strahlung eine elektromagnetische Strahlung ist oder umfasst.

4. Verfahren nach Anspruch 3, wobei die emittierte Strahlung eine Infrarotstrahlung bei einer Spitzenwellenlänge von 805 nm, ist oder umfasst.

5. Verfahren nach einem der Ansprüche 2 bis 4, wobei der das System (1000) verlassende Anteil eine reflektierte, transmittierte oder gestreute Strahlung ist.

6. Verfahren nach einem der vorangegangenen Ansprüche, wobei der Strahlungsempfänger (15) ferner zum Erkennen einer optischen Dichte oder einer Änderung hiervon eingesetzt wird

7. Verfahren nach einem der vorangegangenen Ansprüche, wobei der Fluidfluss des medizinischen Fluids im ersten Förderzustand mittels der Absperreinrichtung (7) angehalten ist, und wobei im zweiten Förderzustand die Fördereinrichtung (5) angehalten ist.

8. Verfahren nach einem der vorangegangenen Ansprüche, wobei als Fördereinrichtung (5) eine Zentrifugalpumpe verwendet wird.

9. Verfahren nach einem der vorangegangenen Ansprüche, wobei ein Alarm ausgegeben wird in dem Fall, dass eine Leckage erkannt wird.

10. System (1000) mit einer Behandlungskassette, mit wenigstens einem ein medizinisches Fluid führenden Abschnitt (100), einer Fördereinrichtung (5) zum Fördern des Fluids durch den Abschnitt (100), einer Absperreinrichtung (7) zum Unterbrechen oder Verringern des Fluidstroms durch den Abschnitt (100), einer Signalsendeeinrichtung zum Emittieren eines Signals, einer Signalempfangseinrichtung zum Empfangen eines Anteils des emittierten Signals und einer Steuereinrichtung, geeignet und konfiguriert zum Ausführen eines Verfahrens nach einem der Ansprüche 1 bis 9.

11. System (1000) nach Anspruch 10, wobei die Signalsendeeinrichtung eine Strahlungsquelle (17) zum Emittieren von Strahlung ist, und wobei die Signalempfangseinrichtung ein Strahlungsempfänger (15) zum Empfangen eines Anteils der emittierten Strahlung ist.

12. System (1000) nach Anspruch 10 oder 11, mit einer Vergleichseinrichtung zum Vergleichen des im ersten Förderzustand empfangenen ersten Anteils des emittierten Signals mit dem im zweiten Förderzustand empfangenen zweiten Anteils des emittierten Signals, und mit einer Entscheidungseinrichtung zum Treffen einer Aussage darüber, ob eine Leckage vorliegt, anhand einer Auswertung des ersten Anteils in Bezug auf den zweiten Anteil.

13. System (1000) nach einem der Ansprüche 10 bis 12, wobei zumindest die Fördereinrichtung (5) Teil der Behandlungskassette ist.

14. System (1000) nach einem der Ansprüche 10 bis 13, wobei die Signalsendeeinrichtung als Strahlungsquelle (17) ausgestaltet ist, um elektromagnetische Strahlung zu emittieren und wobei die Signalempfangseinrichtung ausgestaltet ist, um reflektierte und/oder transmittierte und/oder gestreute Strahlung zu detektieren.

15. Medizintechnische Behandlungsvorrichtung, verbunden mit wenigstens einem System (1000) nach einem der Ansprüche 10 bis 14.

## Claims

1. A method for detecting leakages in a system (1000) conducting a medical fluid, upstream a shut-off device (7) of the system (1000)
- wherein the system (1000) comprises a section (100) conducting the medical fluid, the section (100) being arranged upstream the shut-off device (7);
- wherein the shut-off device (7) is provided or intended for interrupting or reducing an outflow or escaping of the fluid out of the section (100) into an area downstream the shut-off device (7);
- wherein the system (1000) further comprises at least one conveying device (5) for conveying the fluid through the section (100);
wherein the method encompasses the steps of:
- interrupting or reducing the fluid flow through the section (100) or out of the section (100) during a first conveying effort of the conveying device (5) by means of setting or adjusting, respectively, the shut-off device (7) for reaching a first conveying state in the section (100);
- reaching a second conveying state in the section (100) by changing the first conveying effort to a second conveying effort of the conveying device (5);
**characterized in that** the method comprises the following further steps:
- emitting at least one signal by means of a signal emitting device into the section (100) of the system (1000) both in the first and the second conveying state;
- receiving a proportion of the emitted signal leaving the section (100) by means of a signal receiving device in the first conveying state as a first proportion;
- receiving a proportion of the emitted signal leaving the section (100) by means of a signal receiving device in the second conveying state as a second proportion; and
- drawing a conclusion whether a leakage is present by means of an evaluation of the first proportion in relation to the second proportion, wherein drawing the conclusion is or encompasses an evaluation of the first proportion in relation to the second proportion and/or an evaluation of the calculated difference between the first and the second proportion in relation to a threshold value.

2. The method according to claim 1, wherein the emitted signal encompasses or consists of ultrasonic waves and/or radiation, wherein the signal emitting device emits ultrasonic waves and/or is a radiation source (17) for emitting radiation, and wherein the signal receiving device detects ultrasonic waves and/or is a radiation receptor (15) for receiving or detecting radiation.

3. The method according to claim 2, wherein the emitted radiation is or encompasses electromagnetic radiation.

4. The method according to claim 3, wherein the emitted radiation is or encompasses infrared radiation at a peak wavelength of 805 nm.

5. The method according to anyone of the preceding claims 2 to 4, wherein the proportion leaving the system (1000) is reflected, transmitted or scattered radiation.

6. The method according to anyone of the preceding claims, wherein the radiation receptor (15) is further used or applicable or usable or provided or intended for detecting an optical density or a change thereof.

7. The method according to anyone of the preceding claims, wherein the fluid flow of the medical fluid is stopped by means of the shut-off device (7) in the first conveying state and wherein the conveying device (5) is stopped in the second conveying state.

8. The method according to anyone of the preceding claims, wherein a centrifugal pump is used as conveying device (5).

9. The method according to anyone of the preceding claims, wherein an alarm is issued in case a leakage is detected.

10. A system (1000) comprising a treatment cassette, comprising at least one section (100) conducting a medical fluid, a conveying device (5) for conveying the fluid through the section (100), a shut-off device (7) for interrupting or reducing the fluid flow through the section (100), a signal emitting device for emitting a signal, a signal receiving device for receiving a proportion of the emitted signal, and a controller suited and configured for executing a method according to anyone of claims 1 to 9.

11. The system (1000) according to claim 10, wherein the signal emitting device is a radiation source (17) for emitting radiation, and wherein the signal receiving device is a signal receptor (15) for receiving a proportion of the emitted radiation.

12. The system (1000) according to claim 10 or 11, comprising a comparing device for comparing the first proportion of the emitted signal received in the first conveying state and the second proportion of the emitted signal received in the second conveying state, and a decision device for drawing a conclusion whether a leakage is present by means of an evaluation of the first proportion in relation to the second proportion.

13. The system (1000) according to anyone of claims 10 to 12, wherein at least the conveying device (5) is part of the treatment cassette.

14. The system (1000) according to anyone of claims 10 to 13, wherein the signal emitting device is embodied as a radiation source (17) for emitting electromagnetic radiation and wherein the signal receiving device is designed or embodied for detecting reflected and/or transmitted and/or scattered radiation.

15. A medical-technical treatment device being connected with at least one system (1000) according to anyone of claims 10 to 14.

## Revendications

1. Procédé de détection de fuites dans un système (1000) conducteur de fluide médical en amont d'un dispositif de fermeture (7) du système (1000) dans lequel
- le système (1000) comprend une section (100) conductrice de fluide médical située en amont du dispositif de fermeture (7) ;
- le dispositif de fermeture (7) est prévu pour interrompre ou réduire un écoulement du fluide sortant de la section (100) vers une zone avale du dispositif de fermeture (7) ;
- le système (1000) comprend en outre au moins un dispositif de convoyage (5) pour transporter le fluide au travers de la section (100) ;
le procédé comprenant les étapes consistant à :
- interrompre ou diminuer le courant de fluide au travers de la section (100) ou s'écoulant de celle-ci lors d'un premier effort de convoyage du dispositif de convoyage (5) au moyen de l'ajustement du dispositif de fermeture (7) pour obtenir un premier état de convoyage dans la section (100) ;
- obtenir un second état de convoyage dans la section (100) en modifiant le premier effort de convoyage par un deuxième effort de convoyage du dispositif de convoyage (5) ;
**caractérisé en ce que** le procédé comprend les étapes supplémentaires suivantes :
- émettre au moins un signal au moyen d'un dispositif d'émission de signal dans la section (100) du système (1000) tant lors du premier que du deuxième état de convoyage ;
- recevoir une portion du signal émis quittant la section (100) au moyen d'un dispositif de réception de signal lors du premier état de convoyage en tant que première fraction ;
- recevoir une portion du signal émis quittant la section (100) au moyen du dispositif de réception de signal lors du deuxième état de convoyage en tant que deuxième fraction ; et
- conclure si une fuite est présente, sur la base d'une évaluation de la première fraction par rapport à la seconde fraction, la conclusion étant ou comprenant une comparaison de la première fraction par rapport à la seconde fraction et/ou une comparaison de la différence calculée entre la première fraction et la seconde fraction par rapport à une valeur seuil.

2. Procédé selon la revendication 1, dans lequel le signal émis comprend ou consiste d'ondes ultrasonores ou de rayonnement, où le dispositif d'émission de signal émet des ondes ultrasonores et/ou est une source de rayonnement (17) prévue pour émettre des rayonnements, et dans lequel le dispositif de réception de signal détecte des ondes ultrasonores et/ou est un détecteur de rayonnement (15) prévu pour recevoir ou détecter des rayonnements.

3. Procédé selon la revendication 2, dans lequel le rayonnement émis est ou comprend un rayonnement électromagnétique.

4. Procédé selon la revendication 3, dans lequel le rayonnement émis est ou comprend un rayonnement infra-rouge d'une longueur d'onde maximale de 805 nm.

5. Procédé selon l'une quelconque des revendications précédentes 2 à 4, dans lequel la fraction du rayonnement quittant le système (1000) est réfléchie, transmise ou diffusée.

6. Procédé selon l'une quelconque des revendications précédentes, dans lequel le détecteur de rayonnement (15) est en outre utilisé pour détecter une densité optique ou une modification de celle-ci.

7. Procédé selon l'une quelconque des revendications précédentes, dans lequel le courant de fluide médical est stoppé lors du premier état de convoyage au moyen du dispositif de fermeture (7) et dans lequel le dispositif de convoyage (5) est stoppé lors du deuxième état de convoyage.

8. Procédé selon l'une quelconque des revendications précédentes, dans lequel une pompe centrifuge est utilisée comme dispositif de convoyage (5).

9. Procédé selon l'une quelconque des revendications précédentes, dans lequel une alarme est émise dans le cas où une fuite est détectée.

10. Système (1000) avec une cartouche de traitement et comprenant au moins une section (100) conductrice d'un fluide médical, un dispositif de convoyage (5) pour convoyer le fluide au travers de la section (100), un dispositif de fermeture (7) pour interrompre ou réduire le courant de fluide au travers de la section (100), un dispositif d'émission de signal prévu pour émettre un signal, un dispositif de réception de signal prévu pour recevoir une portion du signal émis et un dispositif de commande adapté et configuré pour exécuter un procédé selon l'une quelconque des revendications 1 à 9.

11. Système (1000) selon la revendication 10, dans lequel le dispositif d'émission de signal est une source de rayonnement (17) prévue pour émettre un rayonnement, et dans lequel le dispositif de réception du signal est un détecteur de rayonnement (15) prévu pour recevoir une portion du rayonnement émis.

12. Système (1000) selon la revendication 10 ou 11, avec un dispositif de comparaison prévu pour comparer la première fraction reçue du signal émis lors du premier état de convoyage avec la seconde fraction reçue du signal émis lors du deuxième état de convoyage et comprenant un dispositif d'arbitrage prévu pour conclure si une fuite est présente sur la base d'une évaluation de la première fraction par rapport à la seconde fraction.

13. Système (1000) selon l'une quelconque des revendications 10 à 12, dans lequel au moins le dispositif de convoyage (5) fait partie de la cartouche de traitement.

14. Système (1000) selon l'une quelconque des revendications 10 à 13, dans lequel le dispositif d'émission de signal est réalisé sous la forme d'une source de rayonnement (17) prévue pour émettre un rayonnement électromagnétique et où le dispositif de réception de signal est configuré pour détecter un rayonnement réfléchi et/ou transmis et/ou diffusé.

15. Dispositif médical-technique de traitement relié à au moins un système (1000) selon l'une quelconque des revendications 10 à 14.
